(19) Europäisches Patentamt
European Patent Office
Office européen des brevets

(11) Veröffentlichungsnummer: **0 213 557**
**A2**

(12) # EUROPÄISCHE PATENTANMELDUNG

(21) Anmeldenummer: 86111562.4

(22) Anmeldetag: 21.08.86

(51) Int.Cl.4: C 07 D 317/30
C 07 D 317/24, C 07 D 317/28
C 07 D 319/06, A 01 N 43/28
A 01 N 43/32

(30) Priorität: 30.08.85 DE 3531007

(43) Veröffentlichungstag der Anmeldung:
11.03.87 Patentblatt 87/11

(84) Benannte Vertragsstaaten:
AT BE CH DE FR GB IT LI NL SE

(71) Anmelder: HOECHST AKTIENGESELLSCHAFT
Postfach 80 03 20
D-6230 Frankfurt am Main 80(DE)

(72) Erfinder: Schlegel, Günter, Dr.
Am Flachsland 54
D-6233 Kelkheim (Taunus)(DE)

(72) Erfinder: Mildenberger, Hilmar, Dr.
Fasanenstrasse 24
D-6233 Kelkheim (Taunus)(DE)

(72) Erfinder: Bauer, Klaus, Dr.
Kolpingstrasse 7
D-6054 Rodgau(DE)

(72) Erfinder: Bieringer, Hermann, Dr.
Eichenweg 26
D-6239 Eppstein/Taunus(DE)

(54) **Substituierte Nitrodiphenylether, Verfahren zu ihrer Herstellung und ihre Verwendung als Herbizide.**

(57) Die Verbindungen der Formel (I) oder deren Salze

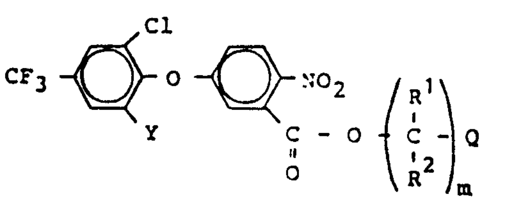

(I)

worin

Q = einen Rest der Formel 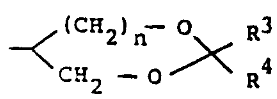

Y = Wasserstoff oder Halogen, $R^1$, $R^2$, $R^3$ = H, (subst.) Alkyl, Alkenyl, Alkinyl, (subst.) Alkoxycarbonyl, (subst.) Phenyl oder $R^1$ und $R^2$ gemeinsam mit dem sie verbindenden C-Atom einen Kohlenwasserstoffring, $R^4$ = H, (subst.) Alkyl, Alkoxycarbonyl oder einen Rest der Formel $R^5OCO-(C_1-C_4)alkyl$, $-COOR^5$ oder $R^1R^5N-CO-(C_1-C_4)$ alkyl und $R^5$ = H, (subst.) Alkyl (subst.) Phenyl oder (subst.) Benzyl; m, n = 0 oder 1 bedeuten mit der Maßgabe, daß für m = 1 n = 0 sein muß und für m = 0 n = 1 sein muß sowie die Verbindungen, in denen beide Reste $R^3$, $R^4$ = Wasserstoff bedeuten, ausgenommen sind, besitzen vorteilhafte herbizide Wirkungen gegen mono- und dikotyle Schadpflanzen.

Croydon Printing Company Ltd.

HOECHST AKTIENGESELLSCHAFT HOE 85/F 168 Dr.AU/St

# Substituierte Nitrodiphenylether, Verfahren zu ihrer Herstellung und ihre Verwendung als Herbizide

Es ist bereits bekannt, daß substituierte Diphenylether herbizide Eigenschaften aufweisen, s. z.B. EP-A 34 883, US-PS 4,358,308, US-PS 4,293,229. Diese weisen jedoch Nachteile bei der Anwendung auf, wie z.B. eine nicht ausreichende Selektivität.

Es wurden nun neue Nitrodiphenylether mit bestimmten Carbonsäureester-Substituenten gefunden, die überraschenderweise eine vorteilhaft selektive Herbizidwirkung besitzen.

Gegenstand der vorliegenden Erfindung sind daher Verbindungen der Formel (I) und deren Salze

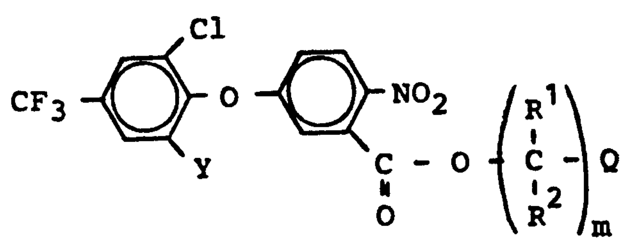

(I)

worin

Q = einen Rest der Formel 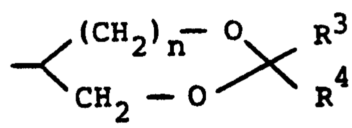

Y = Wasserstoff oder Halogen,

$R^1$,$R^2$,$R^3$= unabhängig voneinander Wasserstoff, $(C_1-C_4)$-Alkyl, das ein- oder mehrfach durch Halogen, Nitro, Cyano, $(C_1-C_4)$Alkoxy, $(C_1-C_4)$Alkylthio, $(C_1-C_4)$Alkoxy)-carbonyl, Di($C_1-C_4$alkyl)-amino, unsubstituiertes oder durch $(C_1-C_4)$Alkyl, $(C_1-C_4)$Alkoxy oder Halogen substituiertes Phenyl, Furanyl oder Thienyl, die beide durch $(C_1-C_4)$Alkyl oder Halogen substituiert sein können, substituiert sein kann,

$(C_2-C_6)$Alkenyl, $(C_2-C_6)$Alkinyl, $(C_1-C_4$-Alkoxy)-carbonyl, das durch unsubstituiertes oder durch $(C_1-C_4)$-Alkyl, $(C_1-C_4)$-Alkoxy, Halogen oder Di$(C_1-C_4$-Alkyl)amino substituiertes Phenyl substituiert sein kann, Phenyl, das durch $(C_1-C_4)$Alkyl, Halogen, Cyano, $(C_1-C_4)$Alkoxy, $(C_1-C_4$-Alkoxy)-carbonyl, Furanyl oder Thienyl, die beide durch $(C_1-C_4)$Alkyl oder Halogen substituiert sein können, substituiert sein kann, oder

$R^1$ und $R^2$ gemeinsam mit dem sie verbindenden C-Atom einen 4- bis 7gliedrigen Kohlenwasserstoffring,

$R^4$= Wasserstoff, $(C_1-C_4)$Alkyl, das durch Halogen, Cyano, $(C_1-C_4)$Alkoxy, $(C_1-C_4)$Alkylthio oder Di$(C_1-C_4$-alkyl)-amino substituiert sein kann, $(C_1-C_4$-Alkoxy)-carbonyl oder einen Rest der Formeln $R^5OCO-(C_1-C_4)$alkyl, $R^1R^5N-CO-(C_1-C_4)$alkyl oder $-COOR^5$,

$R^5$= Wasserstoff, $(C_1-C_4)$Alkyl, das durch Halogen, Cyano, Nitro, $(C_1-C_4)$Alkoxy, $(C_1-C_4)$Alkylthio, unsubstituiertes oder durch $(C_1-C_4)$Alkyl, $(C_1-C_4)$Alkoxy oder Halogen substituiertes Phenyl, Thienyl oder Furanyl, die beide durch $(C_1-C_4)$Alkyl oder Halogen substituiert sein können, substituiert sein kann, unsubstituiertes oder durch $(C_1-C_4)$Alkyl, $(C_1-C_4)$-Alkoxy oder Halogen substituiertes Phenyl oder Benzyl,

m, n= 0 oder 1 bedeuten, mit der Maßgabe, daß a) für m= 1 n= 0 sein muß und b) für m= 0 n= 1 sein muß sowie c) die Verbindungen, in denen beide Reste $R^3$, $R^4$ = Wasserstoff und/oder $(C_1-C_4)$Alkyl bedeuten, ausgenommen sind

$(C_1-C_4)$Alkyl kann in obengenannten Definitionen geradkettig oder verzweigt sein. Halogen bedeutet bevorzugt Fluor, Chlor oder Brom.

Von den Verbindungen der Formel I sind bevorzugt solche, bei denen $R^1$,$R^2$,$R^3$= unabhängig voneinander Wasserstoff oder $(C_1-C_4)$Alkyl, $R^4$= $(C_1-C_4$)Alkoxy)carbonyl, $R^5$OCO-$(C_1-C_4)$alkyl oder $R^1R^5$N-CO-$(C_1-C_4)$-alkyl und $R^5$= $(C_1-C_4)$Alkyl bedeuten.

Im Falle $R^4$= COOH können die Verbindungen der Formel I als Salze vorliegen.

Als landwirtschaftlich einsetzbare Salze für die Verbindung der Formel I kommen beispielsweise infrage: Na-, K-, Mg-, Ca-, Zn-Salze oder Ammonium oder organische mono- bis tetrasubstituierte Ammonium- Sulfonium-, Sulfoxonium- Phosphonium-Salze, wobei als Substituenten beispielsweise Alkyl-, Hydroxyalkyl-, Cycloalkyl-,Phenyl-reste etc. fungieren können.

Die Verbindungen der Formel I besitzen in der Gruppe $-O-CR^1R^2Q$ sowie im Dioxolan- bzw. Dioxan-Rest (Q) selbst optisch aktive Zentren. Sie können daher als Stereoisomerengemische oder in Form der reinen Stereoisomeren vorliegen. Diese werden ebenfalls von vorliegender Erfindung umfaßt. Insbesondere solche Isomeren, die bezüglich des Zentrums $-O-CR^1R^2Q$ optisch einheitlich sind, lassen sich gezielt durch Wahl entsprechend optisch reiner Ausgangsverbindungen synthetisieren.

Gegenstand der vorliegenden Erfindung sind auch Verfahren zur Herstellung der Verbindungen der Formel I und deren Salze dadurch gekennzeichnet, daß man eine Verbindung der Formel (II)

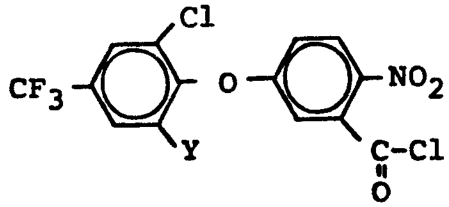

(II)

mit einem Alkohol der Formel (III)

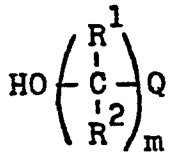

(III)

umsetzt, den dabei freiwerdenden Chlorwasserstoff aus dem Reaktionsgemisch entfernt und die Verbindungen der Formel I gegebenenfalls in ihre Salze überführt.

Der entstehende Chlorwasserstoff kann beispielsweise mit einer üblichen Base wie NaOH, KOH abgefangen werden oder mit Hilfe eines Inertgases ausgetrieben werden.

Die Umsetzung der Verbindungen (II) und (III) erfolgt vorzugsweise in inerten aprotischen Lösungsmitteln, wie z.B. Acetonitril, Dichlormethan, Toluol, Tetrahydrofuran oder Dioxan bei Temperaturen zwischen -20°C und der Siedetemperatur des verwendeten Lösungsmittels, vorzugsweise bei -5°C bis +25°C.

Die Diphenylether-Säurechloride der Formel (II) lassen sich nach bekannten Methoden darstellen (s. z.B. Houben-Weyl, Bd VI/3, S. 85 ff (1965), EP-A 80 312, US-PS 4,031,131).

Die Alkohole der Formel (III) lassen sich nach prinzipiell bekannten Reaktionen darstellen (s. z.B.: J.Org. Chem. 25, 1000 (1960); US-PS 2,500,155).

Die erfindungsgemäßen Verbindungen der Formel I weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf oder Nachauflaufverfahren ausgebracht werden.

Werden die erfindungsgemäßen Verbindungen vor dem Keimen auf die Erdoberfläche appliziert, so wird entweder das Auflaufen der Unkrautkeimlinge vollständig verhindert, oder die Unkräuter wachsen bis zum Keimblattstadium heran, stellen jedoch dann ihr Wachstum ein und sterben schließlich nach Ablauf von drei bis fünf Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit mehr oder weniger schnell ganz ab, so daß auf diese Weise eine für die Kulturpflanzen schädliche Unkrautkonkurrenz sehr früh und nachhaltig durch den Einsatz der neuen erfindungsgemäßen Mittel beseitigt werden kann.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste,

Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Gegenstand der Erfindung sind auch herbizide Mittel, die gekennzeichnet sind durch einen Gehalt an einer Verbindung der Formel I in Kombination mit üblichen Formulierungshilfsmitteln und Inertstoffen sowie deren Verwendung im landwirtschaftlichen Bereich.

Die erfindungsgemäßen Mittel enthalten die Wirkstoffe der Formel I im allgemeinen zu 2-95 Gew.-%. Sie können als Spritzpulver, emulgierbare Konzentrate, versprühbare Lösungen, Stäubemittel oder Granulate in den üblichen Zubereitungen angewendet werden.

Die Spritzpulver sind in Wasser gleichmäßig dispergierbare Präparate, die neben dem Wirkstoff außer einem Verdünnungsmittel oder Inertstoff noch Netzmittel, z.B. polyoxethylierte Alkylphenole, polyoxethylierte Fettalkohole, Alkyl- oder Alkylphenylsulfonate und Dispergiermittel, z.B. ligninsulfonsaures Natrium, 2,2'-dinaphthylmethan-6,6'-disulfonsaures Natrium, dibutylnaphthalinsulfonsaures Natrium oder auch oleylmethyltaurinsaures Natrium enthalten.

Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwandt werden:

Alkylarylsulfonsaure Kalziumsalze wie Ca-dodecyl-benzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyether, Sorbitanfettsäureester, Polyoxethylensorbitfett-säureester oder Polyoxethylensorbitester.

Stäubemittel erhält man durch Vermahlen des Wirkstoffes mit fein verteilten, festen Stoffen, z.B. Talkum, natürlichen Tonen, wie Kaolin, Bentonit, Pyrophillit oder Diatomeenerde.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranalien üblichen Weise - gewünschtenfalls in Mischung mit Düngemitteln - hergestellt werden.

Bei herbiziden Mitteln können die Konzentrationen der Wirkstoffe in den handelsüblichen Formulierungen verschieden sein.

In Spritzpulvern variiert die Wirkstoffkonzentration z.B. zwischen etwa 10 % und 80 %, der Rest besteht aus den oben angegebenen Formulierungszusätzen. Bei emulgierbaren Konzentraten kann die Wirkstoffkonzentration gleichfalls etwa 10 % bis 80 % betragen. Staubförmige Formulierungen enthalten etwa 2- 20 % Wirkstoff. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Zur Anwendung als Herbizide werden die handelsüblichen Konzentrate gegebenenfalls in üblicher Weise verdünnt, z.B. bei Spritzpulvern und emulgierbaren Konzentraten mittels Wasser. Staubförmige und granulierte Zubereitungen sowie versprühbare Lösungen werden vor der Anwendung nicht mehr mit weiteren inerten Stoffen verdünnt. Mit den äußeren Bedingungen wie Temperatur, Feuchtigkeit, u.a. variiert die erforderliche Aufwandmenge. Sie beträgt im allgemeinen zwischen 0.01 und 10 kg/ha, vorzugsweise etwa 0.1 bis 5.0 kg/ha Wirkstoff.

Für einige Anwendungsgebiete kann es zweckmäßig sein, die neuen Herbizide mit einem oder mehreren Herbiziden gemeinsam anzuwenden, z.B. als Tankmischung oder in Form einer Fertigformulierung, um weitere vorteilhafte Wirkungen zu erzielen.

Die erfindungsgemäßen Wirkstoffe können mit anderen Herbiziden, Insektiziden und Fungiziden kombiniert werden.

## Formulierungsbeispiele



### Beispiel 1

Ein Stäubemittel wird erhalten, indem man a) 10 GT (GT= Gewichtsteile) Wirkstoff(e) mit 90 GT Talkum oder einem anderen Inertstoff mischt und in einer Schlagmühle zerkleinert, oder indem man b) 60 GT Wirkstoff, 35 GT Talkum und 5 GT Haftmittel, z.B. ein Polysaccharid, in der gleichen Weise homogenisiert.

### Beispiel 2

Ein in Wasser leicht dispergierbares, benetzbares Pulver wird erhalten, indem man 25 GT Wirkstoff(e) 64 GT kaolinhaltigen Quarz als Inertstoff, 10 GT ligninsulfonsaures Kalium und 1 GT oleoylmethyltaurinsaures Natrium als Netz- und Dispergiermittel mischt und in einer Stiftmühle mahlt. Eine Formulierung mit 5 % Wirkstoffgehalt kann wie folgt zusammengesetzt sein: 5 % Wirkstoff(e), 6 % eines sulfonierten Napthalinformaldehydkondensats (z.B. (R)Dispersogen A der HOECHST AG), 2 % eines Na-Salzes einer Alkylnaphthalinsulfonsäure (z.B. (R)Leonil DB der HOECHST AG), 5 % eines Gemisches aus Polypropylenglykol und $SiO_2$ (z.B. (R)Acrotin 341 der HOECHST AG), 25 % $SiO_2$ und 57 % Kaolin.

### Beispiel 3

Ein in Wasser leicht dispergierbares Dispersionskonzentrat wird erhalten indem man 20 GT Wirkstoff(e) mit 6 GT eines Alkylphenolpolyglykolethers, 3 GT Isotridecanolpolyglykolether (8 Ethylenoxid-Einheiten) und 71 GT paraffinischem Mineralöl (Siedebereich ca. 255 bis über 377°C) mischt und in einer Reibkugelmühle auf eine Feinheit von unter 5 um vermahlt.

### Beispiel 4

Ein emulgierbares Konzentrat wird erhalten aus 15 GT Wirkstoff(e), 75 GT Cyclohexanon als Lösemittel und 10 GT oxethyliertem Nonylphenol (10 Ethylenoxid-Einheiten) als Emulgator.

HERSTELLUNGSBEISPIELE

A. Vorprodukte

Beispiel 1

2-Ethoxycarbonylmethyl-4-hydroxymethyl-1,3-dioxolan

38 g (0.2 mol) 3,3-Diethoxypropionsäureethylester wurden mit 18.4 g (0.2 mol) frisch destilliertem Glycerin und 0,2 g Kaliumhydrogensulfat bei 150°C gerührt, wobei das entstehende Ethanol abdestilliert wurde. Nach beendeter Ethanolentwicklung ließ man abkühlen und destillierte das Produkt im Ölpumpenvakuum am Dünnschichtverdampfer bei 110°C. Man erhielt 16,0 g (42 % d.Th.) 2-Ethoxycarbonylmethyl-4-hydroxymethyl-1,3-dioxolan mit dem Brechungsindes $n_D^{22}$ = 1.458.

Beispiel 2

5-(2-Chlor-4-trifluormethylphenoxy)-2-nitrobenzoesäure

Zu einer Mischung aus 120 ml konz. Schwefelsäure und 200 ml 1,2-Dichlorethan gab man bei 0°C unter kräftigem Rühren 30,0 g (0.095 mol) 3-(2-Chlor-4-trifluormethylphenoxy)-benzoesäure, kühlte auf -10°C und versetzte portionsweise mit 9.5 g (0.095 mol) Kaliumnitrat und 0.5 g Thallium(III)-trifluoracetat.

Nach 0.5 Stunden bei 0°C ließ man das Reaktionsgemisch auf Raumtemperatur erwärmen und goß es anschließend auf 400 ml Eiswasser. Man extrahierte mit Chloroform, trocknete den Extrakt über Natriumsulfat und engte im Vakuum ein.

Man erhielt 27.8 g (81 % d.Th.) 5-(2-Chlor-4-trifluormethylphenoxy)-2-nitrobenzoesäure vom Fp. 150 - 155°C.

0213557

Beispiel 3

5-(2-Chlor-4-trifluormethylphenoxy)-2-nitrobenzoylchlorid

26 g (0.072 mol) 5-(2-Chlor-4-trifluormethylphenoxy)-2-nitrobenzoesäure wurden in 30 ml Thionylchlorid bei einer Badtemperatur von 80°C erhitzt, bis die Gasentwicklung beendet war. Das überschüssige Thionylchlorid wurde im Vakuum abdestilliert und der Rückstand mit n-Hexan behandelt. Nach Filtration wurde 26.2 g (95.8 % d.Th.) 5-(2-Chlor-4-trifluormethyl-phenoxy)-2-nitrobenzoylchlorid vom Fp. 64 - 65°C erhalten.

B. Endprodukte

Beispiel 4

2-Ethoxycarbonylmethyl-1,3-dioxolan-4-yl-methyl-2-nitro-5-(2-chlor-4-trifluormethylphenoxy)-benzoat

Zu einer Lösung von 7.6 g (0.02 mol) 5-(2-Chlor-4-trifluormethylphenoxy)-2-nitrobenzoylchlorid in 20 ml Tetrahydrofuran tropfte man eine auf 0°C gekühlte Mischung aus 3,8 g (0.02 mol) 2-Ethoxycarbonylmethyl-4-hydroxymethyl-1,3-dioxolan und 2.9 ml (0.02 mol) Triethylamin in 20 ml Tetrahydrofuran zu. Nach 2-stündigem Rühren bei 0°C filtrierte man, gab das Filtrat auf Wasser und extrahierte mit Ether. Der getrocknete Etherextrakt wurde eingedampft und chromatographisch über Kieselgel gereinigt. Man erhielt 7.3 g (68 % d.Th.) 2-Ethoxycarbonylmethyl-1,3-dioxolan-4-yl-methyl-2-nitro-5-(2-chlor-4-trifluormethyl-phenoxy)-benzoat als hellgelbes Wachs.

Auf analoge Weise wurden die folgenden Verbindungen der Formel I hergestellt:

Für die Verbindungen 5 - 27 bedeutet m= 1.

Tabelle 1

| Verb.-Nr. | Y | $R^1$ | $R^2$ | Q | Fp. [°C] |
|---|---|---|---|---|---|
| 5 | H | $CH_2Cl$ | H | 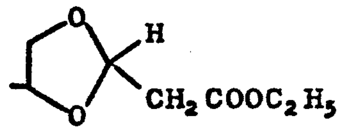 | |
| 6 | H | H | H | 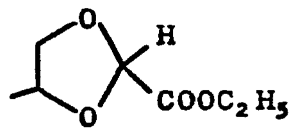 | |
| 7 | H | H | H | 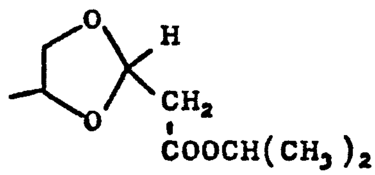 | Wachs |
| 8 | H | H | H | 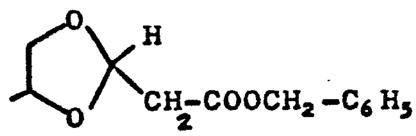 | |
| 9 | H | H | $CH_3$ | 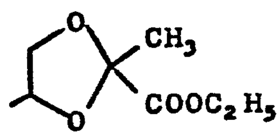 | |
| 10 | H | H | H | 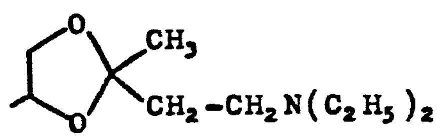 | |
| 11 | H | H | H | 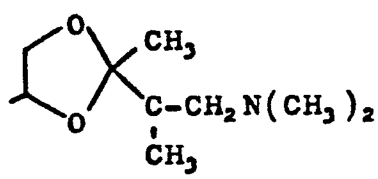 | |
| 12 | H | H | H | 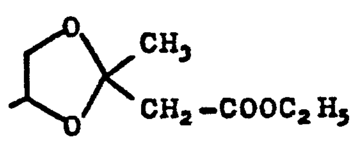 | Wachs |
| 13 | H | H | H | 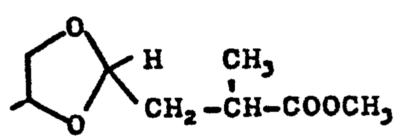 | |

Forts. Tabelle 1

| Verb.-Nr. | Y | $R^1$ | $R^2$ | Q | Fp. [°C] |
|---|---|---|---|---|---|
| 14 | Cl | $C_6H_5$ | H | 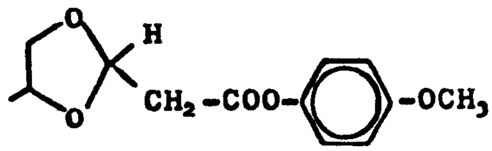 | |
| 15 | Cl | $CH_2CH{=}CH_2$ | H | 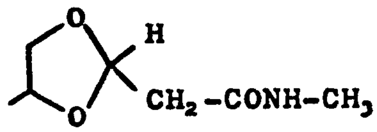 | |
| 16 | Cl | $CH{=}CH_2$ | H | 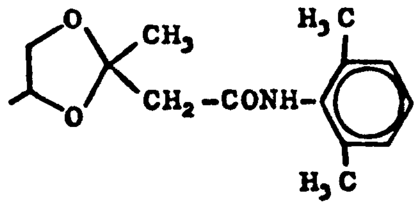 | |
| 17 | Cl | p-$C_6H_4$-$OCH_3$ | H | 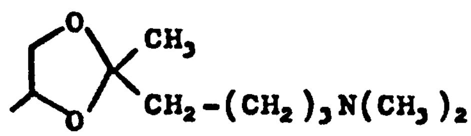 | |
| 18 | Cl | H | H | 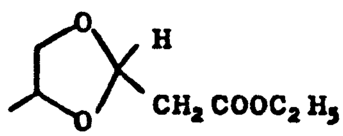 | Wachs |
| 19 | Cl | H | H | 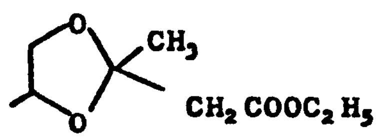 | Wachs |
| 20 | Cl | H | H | 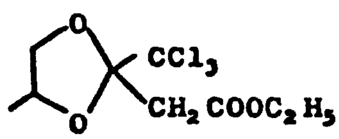 | |
| 21 | Cl | H | H | 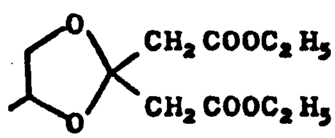 | |
| 22 | Cl | H | H | 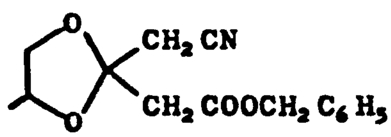 | |

Forts. Tabelle 1

| Verb.-Nr. | Y | $R^1$ | $R^2$ | Q | Fp. [°C] |
|---|---|---|---|---|---|
| 23 | Cl | H | H | 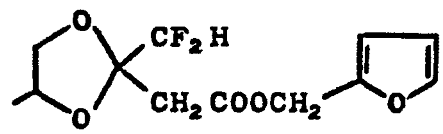 | |
| 24 | H | H | H | 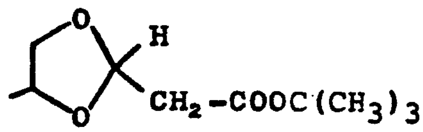 | |
| 25 | Br | H | H | 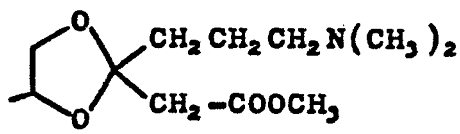 | |
| 26 | F | H | H | 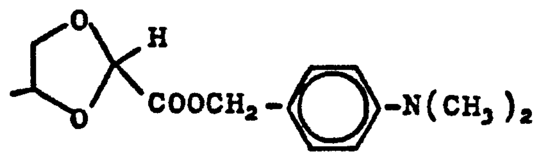 | |

Fortsetzung Tab. 1

| Verb.-Nr. | Y | $R^1$ | $R^2$ | m | Q | Fp(°C) |
|---|---|---|---|---|---|---|
| 27 | H | H | H | 1 | 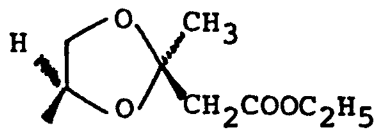 | 68-70 |
| 28 | Cl | H | H | 1 | 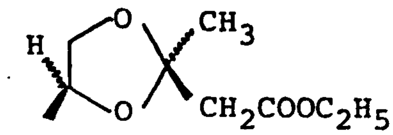 | 85-87 |
| 29 | H | - | - | 0 | 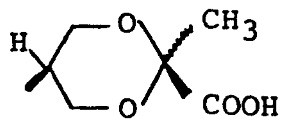 | 60-63 |
| 30 | H | - | - | 0 | 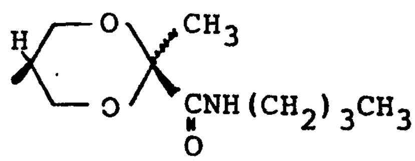 | Wachs |
| 31 | Cl | - | - | 0 | 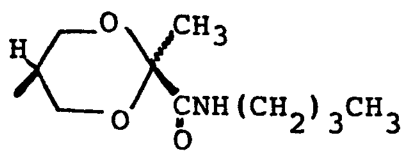 | 41-44 |
| 32 | H | - | - | 0 | 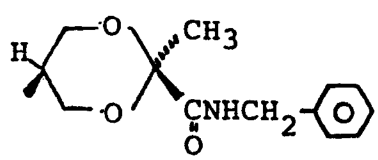 | Wachs |
| 33 | Cl | - | - | 0 | 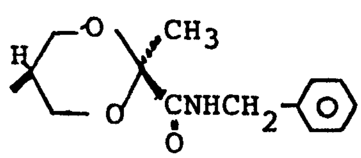 | 49-51 |
| 34 | H | - | - | 0 | 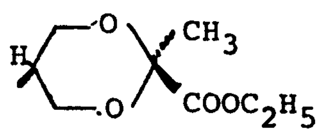 | |
| 35 | F | - | - | 0 | 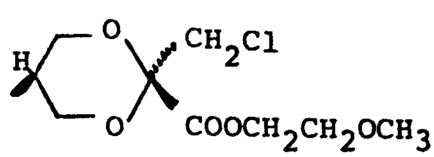 | |

BIOLOGISCHE BEISPIELE

Die Schädigung der Unkrautpflanzen bzw. die Kulturpflanzenverträglichkeit wurde gemäß einem Schlüssel bonitiert, in dem die Wirksamkeit durch Wertzahlen von 0 - 5 ausgedrückt ist. Dabei bedeutet:

| | | |
|---|---|---|
| 0 = | ohne Wirkung bzw. Schaden | |
| 1 = | 0 - 20 % | Wirkung bzw. Schaden |
| 2 = | 20 - 40 % | Wirkung bzw. Schaden |
| 3 = | 40 - 60 % | Wirkung bzw. Schaden |
| 4 = | 60 - 80 % | Wirkung bzw. Schaden |
| 5 = | 80 - 100 % | Wirkung bzw. Schaden |

BEISPIEL 1

Unkrautwirkung im Vorauflauf

Samen bzw. Rhizomstücke von mono- und dikotylen Unkrautpflanzen wurden in Plastiktöpfen (Ø = 9 cm) in sandiger Lehmerde ausgelegt und mit Erde abgedeckt. Die in Form von benetzbaren Pulvern oder Emulsionskonzentraten formulierten erfindungsgemäßen Verbindungen wurden dann als wäßrige Suspensionen bzw. Emulsionen mit einer Wasseraufwandmenge von umgerechnet 600-800 l/ha in unterschiedlichen Dosierungen auf die Oberfläche der Abdeckerde appliziert.

TABELLE 2

Herbizide Wirkung der erfindungsgemäßen Verbindungen im Vorauflauf

| Verb. Nr. | Dosis (Kg a.i./ ha | SIA | CRS | STM | ECG | AS |
|---|---|---|---|---|---|---|
| 12 | 2,5 | 5 | 5 | 4 | 4 | 5 |
| 18 | 2,5 | 5 | 5 | 5 | 5 | 4 |

TABELLE 3

Herbizide Wirkung der erfindungsgemäßen Verbindungen im Nachauflauf

| Verb. Nr. | Dosis (kg a.i./ha) | SIA | CRS | STM | ECG | AS |
|---|---|---|---|---|---|---|
| 12 | 2,5 | 5 | 5 | 5 | 2 | 4 |
| 18 | 2,5 | 5 | 5 | 5 | 4 | 5 |

Abkürzungen Tabelle 2 und 3

SIA = Sinapis arvensis
CRS = Chrysanthemum segetum
STM = Stellaria media
ECG = Echinochloa crus-galli
AS = Avena sativa
a.i. = Aktivsubstanz

PATENTANSPRÜCHE

1. Verbindungen der Formel I und deren Salze

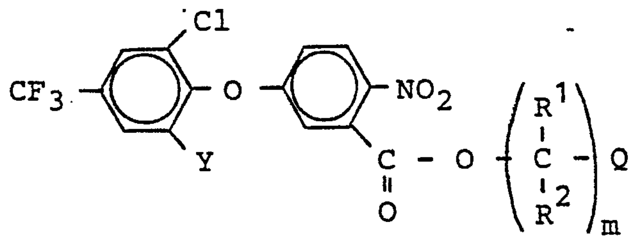

(I)

worin

Q = einen Rest der Formel 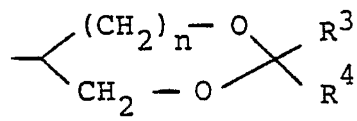

Y = Wasserstoff oder Halogen,

$R^1, R^2, R^3$ = unabhängig voneinander Wasserstoff, $(C_1-C_4)$-Alkyl, das ein- oder mehrfach durch Halogen, Nitro, Cyano, $(C_1-C_4)$Alkoxy, $(C_1-C_4)$Alkylthio, $(C_1-C_4)$Alkoxy)-carbonyl, Di($C_1$-$C_4$alkyl)-amino, unsubstituiertes oder durch $(C_1-C_4)$Alkyl, $(C_1-C_4)$Alkoxy oder Halogen substituiertes Phenyl, Furanyl oder Thienyl, die beide durch $(C_1-C_4)$Alkyl oder Halogen substituiert sein können, substituiert sein kann,

$(C_2-C_6)$Alkenyl, $(C_2-C_6)$Alkinyl, ($C_1$-$C_4$-Alkoxy)-carbonyl, das durch unsubstituiertes oder durch $(C_1-C_4)$-Alkyl, $(C_1-C_4)$-Alkoxy, Halogen oder Di($C_1$-$C_4$-Alkyl)amino substituiertes Phenyl substituiert sein kann, Phenyl, das durch $(C_1-C_4)$Alkyl, Halogen, Cyano, $(C_1-C_4)$Alkoxy, ($C_1$-$C_4$-Alkoxy)-carbonyl, Furanyl oder Thienyl, die beide durch $(C_1-C_4)$Alkyl oder Halogen substituiert sein können, substituiert sein kann,
oder
$R^1$ und $R^2$ gemeinsam mit dem sie verbindenden C-Atom einen 4- bis 7gliedrigen Kohlenwasserstoffring,

$R^4$ = Wasserstoff, $(C_1-C_4)$Alkyl, das durch Halogen, Cyano, $(C_1-C_4)$Alkoxy, $(C_1-C_4)$Alkylthio oder Di$(C_1-C_4$-alkyl)-amino substituiert sein kann, $(C_1-C_4$-Alkoxy)-carbonyl oder einen Rest der Formeln $R^5OCO-(C_1-C_4)$alkyl, $R^1R^5N-CO-(C_1-C_4)$alkyl oder $-COOR^5$,

$R^5$ = Wasserstoff, $(C_1-C_4)$Alkyl, das durch Halogen, Cyano, Nitro, $(C_1-C_4)$Alkoxy, $(C_1-C_4)$Alkylthio, unsubstituiertes oder durch $(C_1-C_4)$Alkyl, $(C_1-C_4)$Alkoxy oder Halogen substituiertes Phenyl, Thienyl oder Furanyl, die beide durch $(C_1-C_4)$Alkyl oder Halogen substituiert sein können, substituiert sein kann, unsubstituiertes oder durch $(C_1-C_4)$Alkyl, $(C_1-C_4)$-Alkoxy oder Halogen substituiertes Phenyl oder Benzyl,

m, n = 0 oder 1 bedeuten, mit der Maßgabe, daß a) für m= 1 n= 0 sein muß und b) für m= 0 n= 1 sein muß sowie c) die Verbindungen, in denen beide Reste $R^3$, $R^4$ = Wasserstoff und/oder $(C_1-C_4)$Alkyl bedeuten, ausgenommen sind.

2. Verbindung der Formel I von Anspruch 1, dadurch gekennzeichnet, daß $R^1$,$R^2$,$R^3$ = unabhängig voneinander Wasserstoff oder $(C_1-C_4)$Alkyl, $R^4$ = $(C_1-C_4$ -Alkoxy)carbonyl, $R^5OCO-(C_1-C_4)$alkyl oder $R^1R^5N-CO-(C_1-C_4)$-alkyl und $R^5$ = $(C_1-C_4)$Alkyl bedeuten.

3. Verfahren zur Herstellung der Verbindungen der Formel I von Ansprüchen 1 oder 2 und ihrer Salze, dadurch gekennzeichnet, daß man eine Verbindung der Formel (II)

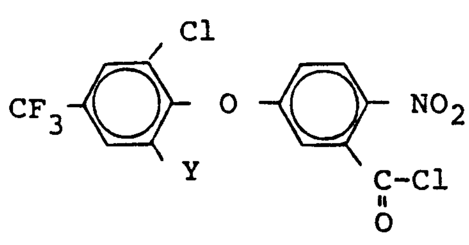

(II)

mit einem Alkohol der Formel (III)

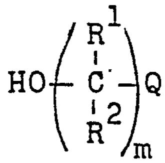 (III)

umsetzt, den dabei freiwerdenden Chlorwasserstoff aus dem Reaktionsgemisch entfernt und die Verbindungen der Formel I gegebenenfalls in ihre Salze überführt.

4. Herbizide Mittel, gekennzeichnet durch einen wirksamen Gehalt an einer Verbindung der Formel I oder deren Salze von Ansprüchen 1 oder 2.

5. Verwendung von Verbindungen der Formel I oder deren Salze von Ansprüchen 1 oder 2 zur Bekämpfung von unerwünschtem Pflanzenwuchs.

6. Verfahren zur Bekämpfung von Schadpflanzen, dadurch gekennzeichnet, daß man auf diese oder auf die von ihnen bewachsenen Böden eine wirksame Menge einer Verbindung der Formel I oder deren Salze von Ansprüchen 1 oder 2 appliziert.

Patentansprüche Österreich:

1. Verfahren zur Herstellung der Verbindungen der Formel I

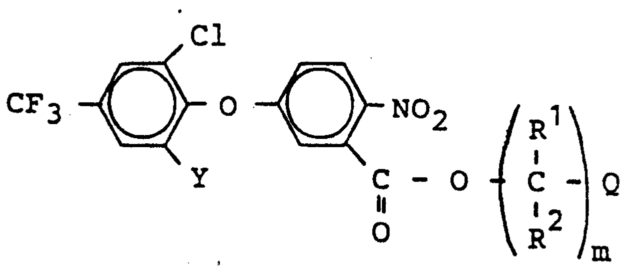

(I)

worin

Q = einen Rest der Formel 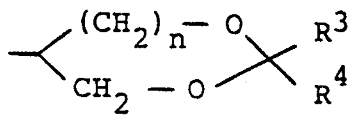

Y = Wasserstoff oder Halogen,

$R^1$, $R^2$, $R^3$ = unabhängig voneinander Wasserstoff, $(C_1-C_4)$-Alkyl, das ein- oder mehrfach durch Halogen, Nitro, Cyano, $(C_1-C_4)$Alkoxy, $(C_1-C_4)$Alkylthio, $(C_1-C_4)$Alkoxy)-carbonyl, Di($C_1-C_4$alkyl)-amino, unsubstituiertes oder durch $(C_1-C_4)$Alkyl, $(C_1-C_4)$Alkoxy oder Halogen substituiertes Phenyl, Furanyl oder Thienyl, die beide durch $(C_1-C_4)$Alkyl oder Halogen substituiert sein können, substituiert sein kann,

$(C_2-C_6)$Alkenyl, $(C_2-C_6)$Alkinyl, ($C_1-C_4$-Alkoxy)-carbonyl, das durch unsubstituiertes oder durch $(C_1-C_4)$-Alkyl, $(C_1-C_4)$-Alkoxy, Halogen oder Di($C_1-C_4$-Alkyl)amino substituiertes Phenyl substituiert sein kann, Phenyl, das durch $(C_1-C_4)$Alkyl, Halogen, Cyano, $(C_1-C_4)$Alkoxy, ($C_1-C_4$-Alkoxy)-carbonyl, Furanyl oder Thienyl, die beide durch $(C_1-C_4)$Alkyl oder Halogen substituiert sein können, substituiert sein kann,
oder
$R^1$ und $R^2$ gemeinsam mit dem sie verbindenden C-Atom einen 4- bis 7gliedrigen Kohlenwasserstoffring,

0213557

$R^4$= Wasserstoff, $(C_1-C_4)$Alkyl, das durch Halogen, Cyano, $(C_1-C_4)$Alkoxy, $(C_1-C_4)$Alkylthio oder Di($C_1-C_4$-alkyl)-amino substituiert sein kann, ($C_1-C_4$-Alkoxy)-carbonyl oder einen Rest der Formeln $R^5OCO-(C_1-C_4)$alkyl, $R^1R^5N-CO-(C_1-C_4)$alkyl oder $-COOR^5$,

$R^5$= Wasserstoff, $(C_1-C_4)$Alkyl, das durch Halogen, Cyano, Nitro, $(C_1-C_4)$Alkoxy, $(C_1-C_4)$Alkylthio, unsubstituiertes oder durch $(C_1-C_4)$Alkyl, $(C_1-C_4)$Alkoxy oder Halogen substituiertes Phenyl, Thienyl oder Furanyl, die beide durch $(C_1-C_4)$Alkyl oder Halogen substituiert sein können, substituiert sein kann, unsubstituiertes oder durch $(C_1-C_4)$Alkyl, $(C_1-C_4)$-Alkoxy oder Halogen substituiertes Phenyl oder Benzyl, m, n = 0 oder 1 bedeuten, mit der Maßgabe, daß a) für m= 1 n= 0 sein muß und b) für m= 0 n= 1 sein muß sowie c) die Verbindungen, in denen beide Reste $R^3$, $R^4$ = Wasserstoff und/oder $(C_1-C_4)$Alkyl bedeuten, ausgenommen sind, dadurch gekennzeichnet, daß man eine Verbindung der Formel II

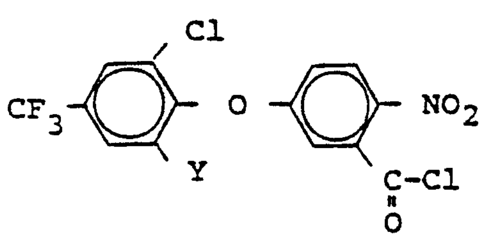

(II)

mit einem Alkohol der Formel (III)

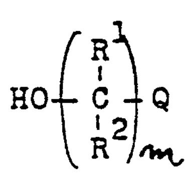

(III)

umsetzt und den dabei freiwerdenden Chlorwasserstoff aus dem Reaktionsgemisch entfernt.

2. Verfahren gemäß Anspruch 1, dadurch gekennzeichnet, daß in Formeln I, II oder III, $R^1$,$R^2$,$R^3$= unabhängig voneinander Wasserstoff oder $(C_1-C_4)$Alkyl, $R^4$= $(C_1-C_4$ -Alkoxy)carbonyl, $R^5$OCO-$(C_1-C_4)$alkyl oder $R^1R^5$N-CO-$(C_1-C_4)$-alkyl und $R^5$= $(C_1-C_4)$Alkyl bedeuten.

3. Herbizide Mittel, gekennzeichnet durch einen wirksamen Gehalt an einer Verbindung der Formel I von Ansprüchen 1 oder 2 oder deren Salze.

4. Verwendung von Verbindungen der Formel I von Ansprüchen 1 oder 2 oder deren Salze zur Bekämpfung von unerwünschtem Pflanzenwuchs.

5. Verfahren zur Bekämpfung von Schadpflanzen, dadurch gekennzeichnet, daß man auf diese oder auf die von ihnen bewachsenen Böden eine wirksame Menge einer Verbindung der Formel I von Ansprüchen 1 oder 2 oder deren Salze appliziert.